# EUROPEAN PATENT SPECIFICATION

(11) **EP 4 389 062 B1**
(45) Date of publication and mention of the grant of the patent: **31.12.2025**
(21) Application number: 23212081.6
(22) Date of filing: 24.11.2023
(51) Int. Cl.: A61C 9/00, A61C 13/00, B23C 1/00

(54) **METHOD FOR PROCESSING DENTAL PROSTHESIS**
VERFAHREN ZUR VERARBEITUNG VON ZAHNPROTHESEN
PROCÉDÉ DE TRAITEMENT DE PROTHÈSE DENTAIRE

(30) Priority: 22.12.2022 KR 20220182024
(43) Date of publication of application: 26.06.2024
(73) Proprietor: Huvitz Co., Ltd., Anyang-si, Gyeonggi-do 14055 (KR)
(72) Inventor: HA, Seung Suk, 14055 Anyang-si (KR); KIM, Beom Mo, 14055 Anyang-si (KR); SHIM, Young Gil, 14055 Anyang-si (KR); KIM, Yong Han, 14055 Anyang-si (KR); LEE, Weon Joon, 14055 Anyang-si (KR)
(74) Representative: Henkel & Partner mbB

(56) References cited:
- US-A1- 2022 338 965

## Description

### FIELD OF THE DISCLOSURE

The present disclosure relates to an apparatus and method for processing a dental prosthesis, and more particularly, to an apparatus and method for processing a dental prosthesis that can reduce the time required for processing work by performing processing preparation work in advance, and can improve convenience by minimizing the movement path of a user.

### BACKGROUND

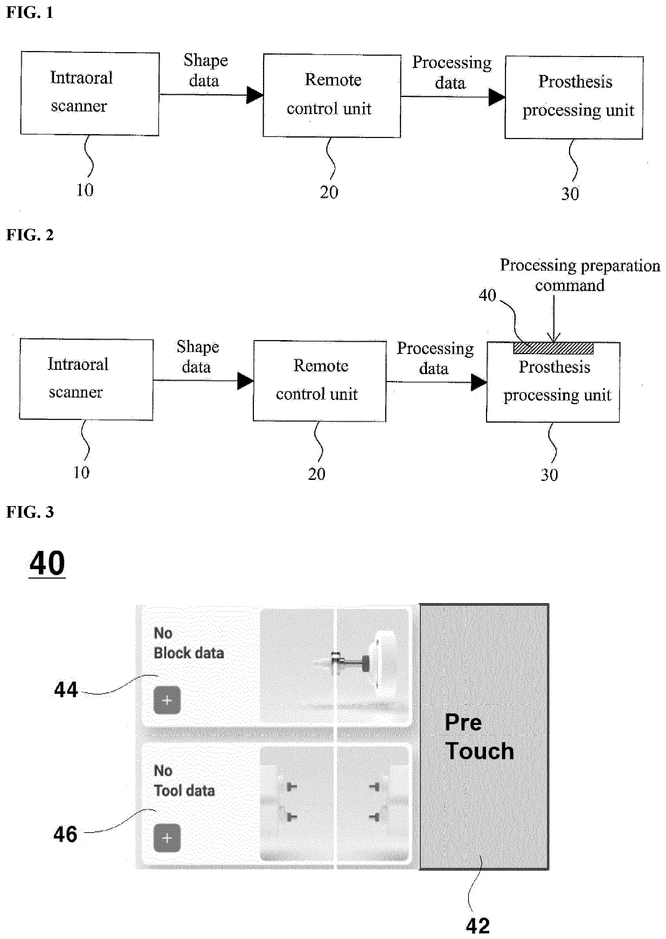

Generally, in order to fabricate a dental prosthesis, oral shape data is obtained by scanning the oral cavity of a patient, processing data for a dental prosthesis is generated from the oral shape data, and then the dental prosthesis is fabricated according to the generated prosthesis processing data. FIG. 1 is a diagram showing a conventional apparatus for processing a dental prosthesis. As shown in FIG. 1, the conventional apparatus for processing a dental prosthesis includes an intraoral scanner 10 that obtains oral shape data by digitally scanning the oral cavity of a patient, a remote control unit 20 including CAD/CAM software that generates processing data for a dental prosthesis from the obtained oral shape data, and a prosthesis processing unit 30 that processes the dental prosthesis according to prosthesis processing data transmitted from the remote control unit 20.

In the conventional apparatus for processing a dental prosthesis shown in FIG. 1, when processing data and processing information are generated in the remote control unit 20 and the generated processing data and processing information are transmitted to the prosthesis processing unit 30, the prosthesis is processed in the prosthesis processing unit 30 according to the transmitted processing data and processing information. When the processing of the prosthesis is completed in the prosthesis processing unit 30, the user moves to the prosthesis processing unit 30 and collects the processed prosthesis. In such a method of processing a prosthesis, since the processing data and processing information are generated in the remote control unit 20, these are all transmitted to the prosthesis processing unit 30, and then processing preparation and processing of the prosthesis are performed in the prosthesis processing unit 30, there exists a problem that the processing of the prosthesis is delayed. Therefore, International Patent Publication WO 2021/058418 discloses a method in which a processing preparation command is transmitted in advance to the prosthesis processing unit 30 and processing preparation for the prosthesis is completed while processing data is being generated in the remote control unit 20, and then the processing data and a processing start command are transmitted from the remote control unit 20 to the prosthesis processing unit 30, thereby reducing the overall working time.

Meanwhile, in a recent apparatus for processing a dental prosthesis, since bidirectional communication is enabled between the remote control unit 20 and the prosthesis processing unit 30, processing data or processing information may be transmitted from the remote control unit 20 to the prosthesis processing unit 30, and the processing data or processing information may be requested from the prosthesis processing unit 30 to the remote control unit 20. At this time, since the remote control unit 20 has a high-performance computing unit, the data transmitted from the remote control unit 20 is not limited by the size of the data, and the data can be used or processed as needed. On the other hand, since the prosthesis processing unit 30 is usually a piece of embedded equipment in which a low-performance computing unit is used, it is difficult to know the shape of the data or work details in advance in the prosthesis processing unit 30. In particular, if a low-performance embedded prosthesis processing unit 30 is used, the user can hardly grasp the shape of the data or work details in the prosthesis processing unit 30. In other words, it is difficult for the low-cost embedded prosthesis processing unit 30 to show CAM data nested with CAD as a 3D image to the user, and even if it is converted into a 2D image, it is difficult to accurately represent the characteristics of the prosthesis due to the nature of the prosthesis. Therefore, the prosthesis processing unit 30 requests processing information from the remote control unit 20 through character or numeri information such as a job number or patient code of work data rather than an image. In this case, there may arise an error that the user incorrectly enters character or numeric information in the prosthesis processing unit 30 and sends incorrect processing information.

US 2022/338965 A1 relates to a method of manufacturing a dental restoration, the method comprising: preparing, in a construction step (S2) through a construction means construction data for a dental tool machine for machining the dental restoration from a workpiece, wherein the construction data defines at least the geometry of the dental restoration within the workpiece, and starting machining, in a starting step (S3), of the dental restoration from the workpiece in accordance with the construction data, further comprising: starting preparing, in a preparing step (S4), before termination of the construction step (S2), the dental tool machine at least through equipping it with all the consumables essential for starting the starting step (S3), and completing preparing the dental tool machine before start of the starting step (S3).

### Prior Art Literature

### Patent Document

1. International Patent Publication WO 2021/058418

### SUMMARY OF THE DISCLOSURE

### TECHNICAL OBJECTS

It is an object of the present disclosure to provide an apparatus and method for processing a dental prosthesis that can reduce the time required for processing work by performing processing preparation work in advance in a prosthesis processing unit in a standby state, i.e., a state where processing work is not ready, and can improve convenience by minimizing the movement path of a user.

It is another object of the present disclosure to provide an apparatus and method for processing a dental prosthesis that can prevent the transmission of incorrect processing data by limiting requests for processing data and processing information in a low-cost embedded prosthesis processing unit.

### TECHNICAL SOLUTION

The present invention provides a method for processing a dental prosthesis in accordance with claim 1. More specifically, the method comprises using an apparatus for processing a dental prosthesis comprising an intraoral scanner (10) configured to obtain oral shape data by scanning an oral cavity of a patient; a remote control unit (20) comprising CAD/CAM software configured to generate processing data for a dental prosthesis from the obtained oral shape data; and a prosthesis processing unit (30) configured to perform processing preparation work for a prosthesis and to process the dental prosthesis according to prosthesis processing data transmitted from the remote control unit (20), and having a user interface (40) through which a processing preparation command for performing the processing preparation work is directly entered, the method comprising: performing processing preparation work in the prosthesis processing unit (30), when a processing preparation command input unit (42) of the user interface (40) mounted on the prosthesis processing unit (30) is activated so that a user can directly enter a processing preparation command into the prosthesis processing unit (30) if processing data has not been transmitted from the remote control unit (20) to the prosthesis processing unit (30), and the user operates the processing preparation command input unit (42) and enters the processing preparation command into the prosthesis processing unit (30); changing the processing preparation command input unit (42) of the user interface (40) into an inactive button (48), when the processing preparation work of the prosthesis processing unit (30) is completed, checking a processing preparation state of the prosthesis processing unit (30) when the processing data has been transmitted from the remote control unit (20) to the prosthesis processing unit (30), a processing start command input unit (52) of the prosthesis processing unit (30) is activated, the user moves from the remote control unit (20) to the prosthesis processing unit (30) and operates the processing start command input unit (52), or when a processing start command is transmitted from the remote control unit (20) to the prosthesis processing unit (30); and starting processing of the dental prosthesis if processing preparation of the prosthesis processing unit (30) has been completed in the checking, wherein the user interface (40) comprises: the processing preparation command input unit (42) configured to allow the user to enter the processing preparation command to cause the prosthesis processing unit (30) to perform the processing preparation work if processing preparation of the prosthesis processing unit (30) has not been completed and the processing data has not been transmitted from the remote control unit (20); a workpiece mounting state check unit (44) configured to provide the user with workpiece mounting state information by displaying a state in which a workpiece to be processed into a prosthesis is mounted on the prosthesis processing unit (30); and a processing tool mounting state check unit (46) configured to provide the user with processing tool mounting state information by displaying a state in which a processing tool for processing the workpiece into a prosthesis of a predetermined shape is mounted on the prosthesis processing unit (30).

### EFFECTS OF THE DISCLOSURE

The method for processing a dental prosthesis in accordance with the present disclosure can reduce the time required for processing work by performing processing preparation work in advance in the prosthesis processing unit in a standby state, i.e., a state where processing work is not ready, and can improve convenience by minimizing the movement path of a user. In addition, according to the method for processing a dental prosthesis in accordance with the present disclosure, the transmission of incorrect processing data can be prevented by limiting requests for processing data and processing information in the low-cost embedded prosthesis processing unit.

### BRIEF DESCRIPTION OF THE DRAWINGS

FIG. 1 is a diagram showing a conventional apparatus for processing a dental prosthesis;
FIG. 2 is a diagram showing one example of an apparatus for processing a dental prosthesis being suitable to be used in a method in accordance with one embodiment of the present disclosure;
FIG. 3 is a diagram showing one example of a user interface (UI) of a prosthesis processing unit to which a method for processing a dental prosthesis in accordance with the present disclosure is applied;
FIG. 4 is a diagram showing a process in which a method for processing a dental prosthesis in accordance with the present disclosure is performed when processing data has not been transmitted to the prosthesis processing unit;
FIG. 5 is a diagram showing the process in which the method for processing a dental prosthesis in accordance with the present disclosure is performed when processing data has been transmitted to the prosthesis processing unit; and
FIG. 6 is a diagram generally showing the process in which the method for processing a dental prosthesis in accordance with the present disclosure is performed.

### DETAILED DESCRIPTION OF THE PREFERRED EMBODIMENTS

Hereinafter, the present disclosure will be described in detail with reference to the accompanying drawings.

FIG. 2 is a diagram showing one example of an apparatus for processing a dental prosthesis in accordance with one embodiment of the present disclosure. As shown in FIG. 2, a prosthesis processing apparatus to which a processing method of the present disclosure can be applied has the same configuration as the conventional one except that the prosthesis processing unit 30 is provided with a user interface 40 through which a processing preparation command for performing processing preparation work is directly entered. Therefore, the prosthesis processing apparatus to which the processing method in accordance with the present disclosure can be applied includes an intraoral scanner 10 that obtains oral shape data by scanning the oral cavity of a patient, a remote control unit 20 including CAD/CAM software that generates processing data for a dental prosthesis from the obtained oral shape data, and a prosthesis processing unit 30 that performs processing preparation work and processes the dental prosthesis according to prosthesis processing data transmitted from the remote control unit 20. The CAD work is to convert the oral shape data into computer-readable design data, and the CAM work is the work of nesting the design data into numerical control data needed for processing a workpiece. The CAD/CAM work requires advanced computations to be performed, and thus is usually performed in the remote control unit 20 equipped with high-performance computing equipment such as a PC provided in the workroom, examination room, or separate space of a user. On the other hand, the prosthesis processing work is performed in the prosthesis processing unit 30 located in a separate work space away from the remote control unit 20 as its working environment is poor, such as the generation of noise and dust.

FIG. 3 is a diagram showing one example of a user interface (UI) 40 of the prosthesis processing unit 30 to which a method for processing a dental prosthesis in accordance with the present disclosure is applied. As shown in FIG. 3, the user interface (UI) 40 of the prosthesis processing unit 30 to which the processing method of the present disclosure can be applied includes a processing preparation command input unit 42, and further includes a workpiece mounting state check unit 44 and a processing tool mounting state check unit 46 as needed. The workpiece mounting state check unit 44 provides the user with workpiece mounting state information by displaying the state in which the workpiece to be processed into a prosthesis (hereinafter referred to as a "block" as necessary) is mounted on the prosthesis processing unit 30, e.g., whether the workpiece is mounted, the material of the mounted workpiece, the position where the workpiece is mounted, and the like. The processing tool mounting state check unit 46 provides the user with processing tool mounting state information by displaying the state in which a processing tool for processing the workpiece into a prosthesis of a predetermined shape is mounted on the prosthesis processing unit 30, e.g., whether the processing tool is mounted, the material or type of the mounted processing tool, the mounting position of the processing tool, and the like. The processing tool may be a conventional cutting tool, an abrasive tool, a drill, or the like. In the present disclosure, the processing tools to be mounted on the prosthesis processing unit 30 may be two or more, preferably four or more, for example, four processing tools with different processing capabilities. In this way, if a number of, e.g., four, different processing tools are mounted on the prosthesis processing unit 30, a variety of types of workpieces can be processed in various ways using combinations of up to four sets of processing tools, and thus it is possible to manufacture prostheses that the user needs without having to separately replace the processing tools.

If the processing preparation of the prosthesis processing unit 30 has not been completed (e.g., in case there is no data on the workpiece mounting state or the processing tool mounting state), and the processing data has not been transmitted from the remote control unit 20 ("No data"), the processing preparation command input unit 42 allows the user to enter a processing preparation command to cause the prosthesis processing unit 30 to perform processing preparation work. In order to process a dental prosthesis in the prosthesis processing unit 30, a variety of processing preparations must usually be performed. Such processing preparations include, for example, (i) checking whether the workpiece (block) is mounted, and measuring the mounting position (length, diameter, mounting rotation angle, etc.) of the workpiece, (ii) checking whether the processing tool is mounted, and measuring the mounting position (length, diameter, etc.) of the processing tool, (iii) whether a processing environment check routine such as checking the water tank, cleaning the water filter, and preheating the cutting tool has been performed, (iv) checking whether the door of the prosthesis processing unit 30 is closed, etc.

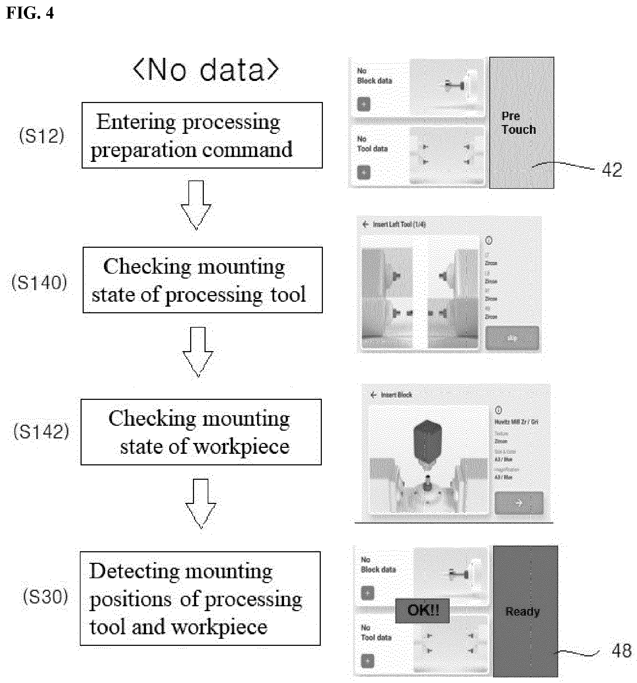

FIG. 4 is a diagram showing a process in which a method for processing a dental prosthesis in accordance with the present disclosure is performed and one example of a user interface screen when processing data has not been transmitted to the prosthesis processing unit 30. As shown in FIG. 4, if processing data has not been transmitted from the remote control unit 20 to the prosthesis processing unit 30 ("No data", "Equipment standby state"), the processing preparation command input unit 42 of the user interface 40 mounted on the prosthesis processing unit 30 is activated so that the user can directly enter a processing preparation command into the prosthesis processing unit 30 (S10, "Pre-touch" state). At this time, information on the workpiece mounting state may be further displayed on the workpiece mounting state check unit 44, and information on the processing tool mounting state may be further displayed on the processing tool mounting state check unit 46. When the user operates the processing preparation command input unit 42 in a method such as touch and enters a processing preparation command into the prosthesis processing unit 30 (S12), the prosthesis processing unit 30 performs processing preparation work (S14). The processing preparation work (S14) includes checking the mounting state of the processing tool (S140), checking the mounting state of the workpiece (block) (S142), and detecting the mounting positions of the processing tool and the workpiece by detecting the position where the processing tool and the workpiece contact each other while moving the processing tool and the workpiece (S30. "Touching control"). The processing preparation work may further include performing a processing environment inspection routine, checking whether the door of the prosthesis processing unit 30 is closed, and the like, as necessary. Further, if the processing tool or workpiece is not mounted, the prosthesis processing unit 30 may display a request for mounting the processing tool or workpiece to the user. In this way, when the processing preparation work of the prosthesis processing unit 30 is completed, the processing preparation command input unit 42 of the user interface 40 mounted on the prosthesis processing unit 30 is changed into an inactive button 48 (Processing preparation is completed, "Ready" state). At this time, information on the mounting states of the workpiece and processing tool may be further displayed on the workpiece mounting state check unit 44 and the processing tool mounting state check unit 46.

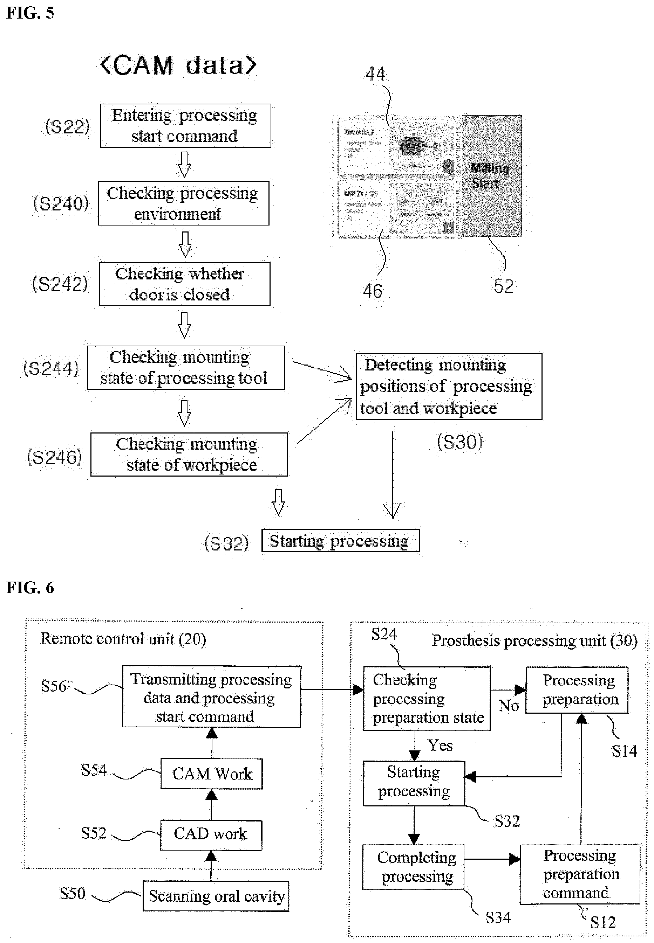

FIG. 5 is a diagram showing the process in which the method for processing a dental prosthesis in accordance with the present disclosure is performed and one example of a user interface screen when processing data has been transmitted to the prosthesis processing unit 30. As shown in FIG. 5, if processing data has been transmitted from the remote control unit 20 to the prosthesis processing unit 30 ("CAM data", "work standby state"), the processing start command input unit 52 ("Milling start") of the user interface 40 mounted on the prosthesis processing unit 30 is activated (S20), and the user operates the processing start command input unit 52 in a method such as touch and enters a processing start command into the prosthesis processing unit 30 (S22), or if a processing start command is transmitted from the remote control unit 20 to the prosthesis processing unit 30, the prosthesis processing unit 30 checks the processing preparation state for the prosthesis (S24). Checking the processing preparation state of the prosthesis processing unit 30 (S24) includes a step of checking the mounting state of the processing tool (S244) and a step of checking the mounting state of the workpiece (S246). In the checking step (S24), if the processing tool and workpiece are not prepared for processing, the processing tool and the workpiece are mounted, the mounting positions of the processing tool and the workpiece are detected by detecting the position where the processing tool and the workpiece contact each other while moving the processing tool and the workpiece (S30. "Touching control"), and a processing preparation step is performed, as necessary. The steps S244 and S246 correspond to steps S140 and S142, respectively. On the other hand, in the checking step (S24), if the processing preparation of the prosthesis processing unit 30, specifically, the processing tool and the workpiece, is completed, processing of the dental prosthesis is started (S32). In addition, checking the processing preparation state (S24) may further include steps such as performing a processing environment inspection routine for the prosthesis processing unit 30 and checking whether the door of the prosthesis processing unit 30 is closed, and if the processing preparation of the above step has not been performed, processing preparation steps such as performing a processing environment inspection routine (S240) and checking whether the door of the prosthesis processing unit 30 is closed (S242) may be further performed.

FIG. 6 is a diagram generally showing the process in which the method for processing a dental prosthesis in accordance with the present disclosure is performed. As shown in FIG. 6, according to the method for processing a dental prosthesis in accordance with the present disclosure, after obtaining oral shape data by scanning the oral cavity of a patient (S50), CAD work for converting the oral shape data into computer-readable design data is performed (S52), and CAM work for nesting the design data into numerical control data needed for processing the workpiece is performed (S54), thereby obtaining prosthesis processing data. The obtained processing data and a processing start command are transmitted to the prosthesis processing unit 30 (S56). When the processing data is transmitted, the processing start command input unit 52 ("Milling start") of the prosthesis processing unit 30 is activated. The user moves from the remote control unit 20 to the prosthesis processing unit 30 and operates the processing start command input unit 52 ("Milling start") (S22), or when a processing start command is transmitted from the remote control unit 20, the step of checking the processing preparation state (S24) is performed. As a result of checking, if the processing preparation is not completed, a processing preparation step (S30, "touching control") for detecting the mounting positions of the processing tool and the workpiece is performed. Here, the user interface (UI) 40 of the prosthesis processing unit 30 guides to perform the processing preparation by displaying unfinished portions of the processing preparation step. When the processing preparation is completed according to the guide, processing of the dental prosthesis is started (S32). When the processing of one dental prosthesis is completed (S34) in this way and the user collects the dental prosthesis, the prosthesis processing unit 30 turns into a state in which processing data has not been transmitted ("No data", "Equipment standby state"), and the processing preparation command input unit 42 of the user interface 40 mounted on the prosthesis processing unit 30 is activated again (S10, "Pre-touch" state). Here, when the user operates the reactivated processing preparation command input unit 42 in a method such as touch and enters a processing preparation command into the prosthesis processing unit 30 (S12), the prosthesis processing unit 30 performs processing preparation work for the next processing (S14).

According to the present disclosure, the processing preparation for the workpiece can be performed in advance in the prosthesis processing unit 30 in the state where processing information for the prosthesis to be processed is not requested from the prosthesis processing unit 30 to the remote control unit 20 and processing data has not been transmitted from the remote control unit 20. Conventionally, the movement path of a user proceeded in the following order: remote control unit 20 → processing preparation of the prosthesis processing unit 30 → CAD/CAM work by the remote control unit 20 → processing in the prosthesis processing unit 30. On the other hand, in the present disclosure, since after completing the processing of one prosthesis in the prosthesis processing unit 30, the processing preparation of the prosthesis processing unit 30 is completed by directly controlling the prosthesis processing unit 30 and it can proceed in the order of the CAD/CAM work by the remote control unit 20 → processing in the prosthesis processing unit 30 in the next work (second prosthesis processing work), the number of movements by the user between the remote control unit 20 and the prosthesis processing unit 30 can be reduced. Generally, in the processing preparation work of the prosthesis processing unit 30, a lot of time is spent in the step (S30) of obtaining the relative position information between the workpiece to be processed and the processing tool. According to the present disclosure, since processing preparation work can be performed in advance if there is no need to replace the processing tool, the processing preparation of the prosthesis processing unit 30 and the CAD/CAM work of the remote control unit 20 can be performed simultaneously, and thus there is an effect of reducing the overall working time.

Although the present disclosure has been described with reference to the accompanying drawings and illustrative embodiments in the above, the present disclosure is not limited to what is shown in the drawings and the embodiments described above. In the following claims, reference numerals are indicated to aid understanding, but the scope of the following claims should not be limited to what is shown by the reference numerals and in the drawings.

## Claims

1. A method for processing a dental prosthesis using an apparatus for processing a dental prosthesis comprising an intraoral scanner (10) configured to obtain oral shape data by scanning an oral cavity of a patient; a remote control unit (20) comprising CAD/CAM software configured to generate processing data for a dental prosthesis from the obtained oral shape data; and a prosthesis processing unit (30) configured to perform processing preparation work for a prosthesis and to process the dental prosthesis according to prosthesis processing data transmitted from the remote control unit (20), and having a user interface (40) through which a processing preparation command for performing the processing preparation work is directly entered, the method comprising:
performing processing preparation work in the prosthesis processing unit (30), when a processing preparation command input unit (42) of the user interface (40) mounted on the prosthesis processing unit (30) is activated so that a user can directly enter a processing preparation command into the prosthesis processing unit (30) if processing data has not been transmitted from the remote control unit (20) to the prosthesis processing unit (30), and the user operates the processing preparation command input unit (42) and enters the processing preparation command into the prosthesis processing unit (30);
changing the processing preparation command input unit (42) of the user interface (40) into an inactive button (48), when the processing preparation work of the prosthesis processing unit (30) is completed,
checking a processing preparation state of the prosthesis processing unit (30) when the processing data has been transmitted from the remote control unit (20) to the prosthesis processing unit (30), a processing start command input unit (52) of the prosthesis processing unit (30) is activated, the user moves from the remote control unit (20) to the prosthesis processing unit (30) and operates the processing start command input unit (52), or when a processing start command is transmitted from the remote control unit (20) to the prosthesis processing unit (30); and
starting processing of the dental prosthesis if processing preparation of the prosthesis processing unit (30) has been completed in the checking,
wherein the user interface (40) comprises:
the processing preparation command input unit (42) configured to allow the user to enter the processing preparation command to cause the prosthesis processing unit (30) to perform the processing preparation work if processing preparation of the prosthesis processing unit (30) has not been completed and the processing data has not been transmitted from the remote control unit (20);
a workpiece mounting state check unit (44) configured to provide the user with workpiece mounting state information by displaying a state in which a workpiece to be processed into a prosthesis is mounted on the prosthesis processing unit (30); and
a processing tool mounting state check unit (46) configured to provide the user with processing tool mounting state information by displaying a state in which a processing tool for processing the workpiece into a prosthesis of a predetermined shape is mounted on the prosthesis processing unit (30).

2. The method for processing a dental prosthesis of claim 1, wherein when processing of one dental prosthesis is completed and the user collects the dental prosthesis, the prosthesis processing unit (30) turns into in a state in which processing data has not been transmitted, and the processing preparation command input unit (42) of the user interface (40) mounted on the prosthesis processing unit (30) is activated again.

3. The method for processing a dental prosthesis of claim 2, wherein when the user operates the processing preparation command input unit (42) which is activated again and enters a processing preparation command into the prosthesis processing unit (30), the prosthesis processing unit (30) performs processing preparation work for the next processing.

## Patentansprüche

1. Verfahren zur Bearbeitung einer Zahnprothese unter Verwendung einer Vorrichtung zur Bearbeitung einer Zahnprothese, die einen intraoralen Scanner (10), der so konfiguriert ist, dass er durch Scannen der Mundhöhle eines Patienten Daten zur Mundform erhält; eine Fernsteuereinheit (20), die eine CAD/CAM-Software umfasst, die so konfiguriert ist, dass sie aus den erhaltenen Daten zur Mundform Bearbeitungsdaten für eine Zahnprothese generiert; und eine Einheit (30) zur Bearbeitung von Prothesen, die so konfiguriert ist, dass sie Bearbeitungsvorbereitungsarbeiten für eine Prothese durchführt und die Zahnprothese entsprechend den von der Fernsteuereinheit (20) übermittelten Prothesenbearbeitungsdaten bearbeitet, und die eine Benutzerschnittstelle (40) aufweist, über die ein Bearbeitungsvorbereitungsbefehl zur Durchführung der Bearbeitungsvorbereitungsarbeiten direkt eingegeben wird, umfasst, wobei das Verfahren umfasst:
Durchführen von Bearbeitungsvorbereitungsarbeiten in der Einheit (30) zur Bearbeitung von Prothesen, wenn eine Einheit (42) zur Eingabe eines Bearbeitungsvorbereitungsbefehls der an der Einheit (30) zur Bearbeitung von Prothesen montierten Benutzerschnittstelle (40) aktiviert wird, so dass ein Benutzer einen Bearbeitungsvorbereitungsbefehl in die Einheit (30) zur Bearbeitung von Prothesen eingeben kann, wenn keine Bearbeitungsdaten von der Fernsteuereinheit (20) an die Einheit (30) zur Bearbeitung von Prothesen übermittelt wurden, und der Benutzer die Einheit (42) zur Eingabe eines Bearbeitungsvorbereitungsbefehls bedient und den Befehl zur Bearbeitungsvorbereitung in die Einheit (30) zur Bearbeitung von Prothesen eingibt;
Ändern der Einheit (42) zur Eingabe eines Bearbeitungsvorbereitungsbefehl der Benutzerschnittstelle (40) in eine inaktive Taste (48), wenn die Bearbeitungsvorbereitungsarbeiten der Einheit (30) zur Bearbeitung von Prothesen abgeschlossen sind,
Überprüfen eines Bearbeitungsvorbereitungszustands der Einheit (30) zur Bearbeitung von Prothesen, wenn die Bearbeitungsdaten von der Fernsteuereinheit (20) an die Einheit (30) zur Bearbeitung von Prothesen übermittelt wurden, eine Einheit (52) zur Eingabe eines Bearbeitungsstartbefehls der Einheit (30) zur Bearbeitung von Prothesen aktiviert wird, der Benutzer von der Fernsteuereinheit (20) zu der Einheit (30) zur Bearbeitung von Prothesen wechselt und die Einheit (52) zur Eingabe eines Bearbeitungsstartbefehls betätigt oder wenn ein Bearbeitungsstartbefehl von der Fernsteuereinheit (20) an die Einheit (30) zur Bearbeitung von Prothesen übermittelt wird; und
Starten der Bearbeitung der Zahnprothese, wenn die Bearbeitungsvorbereitung der Einheit (30) zur Bearbeitung von Prothesen bei der Überprüfung abgeschlossen wurde, wobei die Benutzerschnittstelle (40) umfasst:
die Einheit (42) zur Eingabe eines Bearbeitungsvorbereitungsbefehls, die so konfiguriert ist, dass sie es dem Benutzer ermöglicht, den Bearbeitungsvorbereitungsbefehl einzugeben, um zu bewirken, dass die Einheit (30) zur Bearbeitung von Prothesen die Bearbeitungsvorbereitungsarbeiten durchführt, wenn die Bearbeitungsvorbereitung der Einheit (30) zur Bearbeitung von Prothesen nicht abgeschlossen ist und die Bearbeitungsdaten nicht von der Fernsteuereinheit (20) übermittelt wurden;
eine Einheit (44) zur Überprüfung des Montagezustands eines Werkstücks, die so konfiguriert ist, dass sie dem Benutzer Informationen über den Montagezustand des Werkstücks bereitstellt, indem sie einen Zustand anzeigt, in dem ein Werkstück, das zu einer Prothese verarbeitet werden soll, an der Einheit (30) zur Bearbeitung von Prothesen montiert ist; und
eine Einheit (46) zur Überprüfung des Montagezustands eines Bearbeitungswerkzeugs, die so konfiguriert ist, dass sie dem Benutzer Informationen über den Montagezustand des Bearbeitungswerkzeugs bereitstellt, indem sie einen Zustand anzeigt, in dem ein Bearbeitungswerkzeug zur Bearbeitung des Werkstücks zu einer Prothese mit einer vorgegebenen Form an der Einheit (30) zur Bearbeitung von Prothesen montiert ist.

2. Verfahren zur Bearbeitung einer Zahnprothese nach Anspruch 1, wobei, wenn die Bearbeitung einer Zahnprothese abgeschlossen ist und der Benutzer die Zahnprothese entnimmt, die Einheit (30) zur Bearbeitung von Prothesen in einen Zustand übergeht, in dem keine Bearbeitungsdaten übermittelt wurden, und die Einheit (42) zur Eingabe eines Bearbeitungsvorbereitungsbefehls, die an der Einheit (30) zur Bearbeitung von Prothesen montiert ist, wieder aktiviert wird.

3. Verfahren zur Bearbeitung einer Zahnprothese nach Anspruch 2, wobei, wenn der Benutzer die wieder aktivierte Einheit (42) zur Eingabe eines Bearbeitungsvorbereitungsbefehls betätigt und einen Bearbeitungsvorbereitungsbefehl in die Einheit (30) zur Bearbeitung von Prothesen eingibt, die Einheit (30) zur Bearbeitung von Prothesen die Bearbeitungsvorbereitungsarbeiten für die nächste Bearbeitung durchführt.

## Revendications

1. Procédé de traitement d'une prothèse dentaire à l'aide d'un appareil de traitement d'une prothèse dentaire comprenant un scanner intra-oral (10) configuré pour obtenir des données de forme buccale par balayage d'une cavité buccale d'un patient ; une unité de commande à distance (20) comprenant un logiciel CAO/FAO configuré pour générer des données de traitement pour une prothèse dentaire à partir des données de forme buccale obtenues ; et une unité de traitement de prothèse (30) configurée pour effectuer un travail de préparation de traitement pour une prothèse et pour traiter la prothèse dentaire en fonction des données de traitement de prothèse transmises par l'unité de commande à distance (20), et présentant une interface utilisateur (40) par l'intermédiaire de laquelle une commande de préparation de traitement pour effectuer le travail de préparation de traitement est directement entrée, le procédé comprenant : l'exécution d'un travail de préparation de traitement dans l'unité de traitement de prothèse (30), lorsqu'une unité d'entrée de commande de préparation de traitement (42) de l'interface utilisateur (40) montée sur l'unité de traitement de prothèse (30) est activée de sorte qu'un utilisateur puisse directement entrer une commande de préparation de traitement dans l'unité de traitement de prothèse (30) si des données de traitement n'ont pas été transmises de l'unité de commande à distance (20) à l'unité de traitement de prothèse (30), et l'utilisateur actionne l'unité d'entrée de commande de préparation de traitement (42) et entre la commande de préparation de traitement dans l'unité de traitement de prothèse (30) ; la transformation de l'unité d'entrée de commande de préparation de traitement (42) de l'interface utilisateur (40) en un bouton inactif (48), lorsque le travail de préparation de traitement de l'unité de traitement de prothèse (30) est terminé, la vérification d'un état de préparation de traitement de l'unité de traitement de prothèse (30) lorsque les données de traitement ont été transmises de l'unité de commande à distance (20) à l'unité de traitement de prothèse (30), une unité d'entrée de commande de démarrage de traitement (52) de l'unité de traitement de prothèse (30) est activée, l'utilisateur se déplace de l'unité de commande à distance (20) à l'unité de traitement de prothèse (30) et actionne l'unité d'entrée de commande de démarrage de traitement (52), ou lorsqu'une commande de démarrage de traitement est transmise de l'unité de commande à distance (20) à l'unité de traitement de prothèse (30) ; et le démarrage du traitement de la prothèse dentaire si la préparation de traitement de l'unité de traitement de prothèse (30) a été achevée lors de la vérification, dans lequel l'interface utilisateur (40) comprend : l'unité d'entrée de commande de préparation de traitement (42) configurée pour permettre à l'utilisateur d'entrer la commande de préparation de traitement pour amener l'unité de traitement de prothèse (30) à effectuer le travail de préparation de traitement si la préparation de traitement de l'unité de traitement de prothèse (30) n'a pas été achevée et que les données de traitement n'ont pas été transmises par l'unité de commande à distance (20) ;
une unité de vérification d'état de montage de pièce (44) configurée pour fournir à l'utilisateur des informations d'état de montage de pièce en affichant un état dans lequel une pièce à traiter en une prothèse est montée sur l'unité de traitement de prothèse (30) ; et
une unité de vérification d'état de montage d'outil de traitement (46) configurée pour fournir à l'utilisateur des informations d'état de montage d'outil de traitement en affichant un état dans lequel un outil de traitement pour traiter la pièce en une prothèse d'une forme prédéterminée est monté sur l'unité de traitement de prothèse (30).

2. Procédé de traitement d'une prothèse dentaire selon la revendication 1, dans lequel, lorsque le traitement d'une prothèse dentaire est terminé et que l'utilisateur recueille la prothèse dentaire, l'unité de traitement de prothèse (30) se met dans un état dans lequel les données de traitement n'ont pas été transmises, et l'unité d'entrée de commande de préparation de traitement (42) de l'interface utilisateur (40) montée sur l'unité de traitement de prothèse (30) est à nouveau activée.

3. Procédé de traitement d'une prothèse dentaire selon la revendication 2, dans lequel lorsque l'utilisateur actionne l'unité d'entrée de commande de préparation de traitement (42) qui est de nouveau activée et entre une commande de préparation de traitement dans l'unité de traitement de prothèse (30), l'unité de traitement de prothèse (30) effectue un travail de préparation de traitement pour le traitement suivant.
